# EUROPÄISCHE PATENTSCHRIFT

(11) **EP 0 803 240 B1**
(45) Veröffentlichungstag und Bekanntmachung des Hinweises auf die Patenterteilung: **19.06.2002**
(21) Anmeldenummer: 97250106.8
(22) Anmeldetag: 07.04.1997
(51) Int. Cl.: A61K 6/083

(54) **Gefülltes und polymerisierbares Dentalmaterial**
Polymerisable dental material containing a filler
Matériau dentaire polymérisable contenant une charge

(30) Priorität: 26.04.1996 DE 19617931
(43) Veröffentlichungstag der Anmeldung: 29.10.1997
(73) Patentinhaber: Ivoclar Vivadent AG, 9494 Schaan (LI)
(72) Erfinder: Rheinberger, Volker, Dr., 9490 Vaduz (LI); Moszner, Norbert, Prof.-Dr., 9492 Eschen (LI); Fischer, Urs Karl, 9320 Arbon (CH)
(74) Vertreter: UEXKÜLL & STOLBERG

(56) Entgegenhaltungen:
- EP-A- 0 315 186
- EP-A- 0 368 657
- GB-A- 1 596 241
- I. KREJCI AND F. LUTZ: "TULUX-Zement; Ein neuer, zweizeitig lichthärtender Kompositzement für adhäsiv verankerte Restaurationen" SCHWEIZ. MONATSSCHR. ZAHNMED., Bd. 99, Nr. 4, April 1989, BERN CH, Seiten 454-461, XP002057821

## Beschreibung

Die Erfindung betrifft ein gefülltes und polymerisierbares Dentalmaterial, welches insbesondere in Form von Füllungskompositen, Befestigungszementen oder Adhäsiven eingesetzt werden kann.

Die Eigenschaften von dentalen Füllungskompositen sind außer von der Struktur der organischen Matrix auch von den Eigenschaften der eingesetzten Füllstoffe abhängig. Wesentlichen Einfluß auf die Gesamteigenschaften des Komposits haben dabei Teilchengröße, Größenverteilung, Teilchenform, Art der Teilchenoberfläche, chemische Zusammensetzung, Art einer etwaigen Oberflächenmodifizierung und optische Eigenschaften der Füllstoffe sowie der Gesamtgehalt an Füllstoffen (vgl. J.F. Roulet, Degradation of Dental Polymers, Karger, Basel 1987, Seite 10).

Eine hohe Festigkeit der dentalen Füllungskomposite sowie ein geringer Polymerisationsschrumpf können vor allem durch einen hohen Füllungsgrad erreicht werden. Dabei wird der optimale Füllungsgrad auch durch die erforderliche Konsistenz der in der Praxis eingesetzten Kompositpasten bestimmt. Diese müssen so beschaffen sein, daß ein optimales Einbringen der Materialien in die Zahnkavität sowie ein optimales Verarbeiten möglich ist. Es ist bekannt, daß durch die Zugabe von Füllstoffen zu der Matrixmonomermischung die Viskosität mit dem Füllstoffgehalt ansteigt, wobei die Verdickungswirkung z.B. der hochdispersen Kieselsäure, die nach der DE-C-24 03 211 als Füllstoff in Dentalmassen Anwendung findet, mit abnehmender Primärpartikelgröße und mit steigender BET-Oberfläche zunimmt. Dementsprechend zeichnen sich gegenwärtig verwendete Mikrofüller-Komposite durch einen Füllungsgrad mit anorganischen Füllstoffen von ca. 50 Gew.-% und Hybrid-Komposite durch einen Füllungsgrad von ca. 80 Gew.-% aus.

Demgegenüber sind im Falle von Befestigungszementen auf Kompositbasis im Vergleich zu den entsprechenden permanenten Füllungskompositen geringere Viskositäten erforderlich, so daß auch nur ein geringerer Füllungsgrad erzielt werden kann. Für Dentaladhäsive ist in der Regel eine dünnflüssige Konsistenz erforderlich, weshalb bei diesen auf die Zugabe von Füllstoffen in der Regel völlig verzichtet werden muß.

Die Verwendung von Produkten von Sol-Gel-Verfahren als Bestandteil von Dentalmaterialien ist bekannt. So werden in DE-C-39 13 252, EP-B-394 794 und EP-B-523 545 Dentalmaterialien beschrieben, die als feinteiligen Füllstoff Heterosiloxane enthalten. Die eingesetzten Heterosiloxane werden durch Cokondensation von entsprechenden Silanen mit Metallalkoxiden als statistische Copolykondensate, Block-Copolykondensate oder sogenannte gemischte Copolykondensate hergestellt. Dabei ist es erforderlich, daß die nach Durchführung des Sol-Gel-Prozesses anfallenden Feststoffe abgetrennt, gewaschen, getrocknet, temperiert gemahlen und unter Umständen auch noch durch Oberflächensilanisierung funktionalisiert werden. Diese Prozeßstufen beeinflussen die Eigenschaften des schließlich erhaltenen Füllstoffes dergestalt, daß dieser lediglich in agglomerierter Form vorliegt.

Analoge Sol-Gel-Füllstoffe auf Basis von Heteropolysiloxanen können gemäß EP-B-381 961 für dentale Füllungsmaterialien eingesetzt werden.

Weiter sind aus der DE-A-41 33 494 Dentalmaterialien auf der Basis von polymerisierbaren Polysiloxanen bekannt, die ebenfalls durch den Sol-Gel-Prozeß von hydrolytisch kondensierbaren Silanen erhalten werden. Die nach der Kondensation erhaltenen Harze sind hochviskos und können kaum weiter gefüllt werden.

Aus der WO 92/16183 sind Zusammensetzungen auf der Basis von organisch modifizierten Kieselsäure-Polykondensaten bekannt, welche sich zur Beschichtung von Zähnen und Zahnersatzteilen einsetzen lassen. Dabei ist es erforderlich, daß die erhaltenen anorganisch-organischen Vorkondensate zur Einstellung der Viskosität der Zusammensetzungen mit einem Lösungsmittel verdünnt werden.

Die GB-A-2 257 438 offenbart Produkte des Sol-Gel-Prozesses zur glasartigen Beschichtung von Zähnen.

Die EP 0 315 186 A2 offenbart Dentalmaterialien, die als Füllstoff hydrophobe Silicapartikel mit einem Durchmesser von 0,01 bis 0,04 µm enthalten. Der Füllstoff ist in einem Harz auf der Basis von ethoxyliertem Bisphenol-A-dimethacrylat dispergiert.

Die GB-A-1 596 241 betrifft Zusammensetzungen zur Versiegelung von Fissuren und Poren in Zahnschmelz, die ein polymerisierbares Harz, opaken Füllstoff und ein Suspensionsmittel, wie beispielsweise koaguliertes Kieselgel, enthalten.

Die EP 0 368 657 A2 offenbart Dentalmaterialien, die einen feinteiligen, wasserunlöslichen Füllstoff mit einer Größe von 0.1 µm oder weniger enthalten, der mit einem Silanhaftvermittler oberflächenbehandelt ist, welcher eine (Meth)acryloylgruppe und eine lineare Alkylengruppe mit mindestens 8 Kohlenstoffatomen aufweist.

Schließlich ist es auch bekannt, daß die Herstellung von SiO₂-Solen durch Hydrolyse und Kondensation von geeigneten Vorläufern, wie z.B. kondensierbaren Silanen, möglich ist. Dabei hängt die Sol-Partikelbildung unter anderem von der Art der Vorläufer, der Art des Reaktionsmediums, dem pH-Wert, dem Katalysator oder der eingesetzten Wassermenge ab (vgl. C.J. Brinker, G.W. Scherer, Sol-Gel-Science, Academic Press, Boston 1990, Seiten 99 ff. und 617 ff.).

Bei Kieselsolen handelt es sich um wäßrige Lösungen von kolloidalem, amorphen SiO₂, welche in der Regel 10 bis 50 Gew.-% SiO₂-Teilchen mit einem Durchmesser von 5 bis 150 nm enthalten (vgl. Ullmann's Encyklopädie der technischen Chemie, 4. Auflage, Band 21, Verlag Chemie, Weinheim 1982, Seiten 456 ff.). Dabei ist es möglich, die Teilchen von diesen wäßrigen Kieselsolen z.B. mit 3-(Methacryloyloxy)-propyltrimethoxysilan zu silanisieren und einen Lösungsmittelaustausch, z.B. mit Isopropanol oder Monomer, durchzuführen (vgl. L.N. Lewis et al., Polym. Mat. Sci. Techn., Proceed. Amer. Chem. Soc., 72 (1995), Seite 583).

Der Erfindung liegt nunmehr die Aufgabe zugrunde, ein gefülltes und polymerisierbares Dentalmaterial zur Verfügung zu stellen, welches im Vergleich zu Materialien mit konventionellen Füllstoffen eine geringere Viskosität, verbesserte mechanische Eigenschaften sowie einen verringerten Polymerisationsschrumpf zeigt.

Diese Aufgabe wird durch das erfindungsgemäße gefüllte und polymerisierbare Dentalmaterial nach den Ansprüchen 1 bis 12 gelöst.

Weiter betrifft die Erfindung ebenfalls die Verwendung eines gefüllten und polymerisierbaren Materials als Dentalmaterial nach Anspruch 13.

Das erfindungsgemäße gefüllte und polymerisierbare Dentalmaterial zeichnet sich dadurch aus, daß es
(a) ein Sol von amorphen SiO₂-Teilchen in einem flüssigen, organischen Dispersionsmittel enthält, wobei die SiO₂-Teilchen organisch oberflächenmodifiziert sind, eine mittlere Größe von 10 bis 100 nm aufweisen und nichtagglomeriert sind.

Daß die SiO₂-Teilchen nicht-agglomerisiert vorliegen, ist z.B. mittels Transmissionselektronenmikroskopie (TEM) nachweisbar. Ebenfalls mittels TEM wird die mittlere Größe der Teilchen bestimmt.

Im folgenden wird das Sol (a) auch als ein Kieselsäureorganosol bezeichnet.

Das erfindungsgemäße Dentalmaterial enthält üblicherweise 1 bis 50 Gew.-% des Soles (a). Der Gehalt des Soles (a) an SiO₂-Teilchen beträgt üblicherweise 10 bis 70 Gew.-% und insbesondere 20 bis 55 Gew.-%, bezogen auf das Sol.

Die SiO₂-Teilchen des Soles (a) liegen in einem flüssigen, organischen Dispersionsmittel vor. Bevorzugt enthält das flüssige, organische Dispersionsmittel mindestens ein Diol, mindestens ein Hydroxy(meth)acrylat, mindestens ein Di(meth)acrylat oder Mischungen der vorstehenden Verbindungen. Besonders bevorzugt sind solche flüssigen, organischen Dispersionsmittel, die Hexandioldiacrylat, 2-Hydroxyethylmethacrylat, Triethylenglycoldimethacrylat, Bisphenol-A-Glycidylmethacrylat, ein Urethandimethacrylat oder Mischungen der vorstehenden Verbindungen enthalten. Besonders vorteilhafte Mischungen von flüssigem, organischen Dispersionsmittel sind Mischungen von Triethylenglycoldimethacrylat (TEGDMA), Bisphenol-A-Glycidylmethacrylat und dem Urethandimethacrylat aus 2-Hydroxyethylmethacrylat (HEMA) und 2,2,4-Trimethylhexamethylendiisocyanat (TMDI). Es ist bevorzugt, daß das Dispersionsmittel mindestens eine polymerisierbare Verbindung enthält.

Die SiO₂-Teilchen des Kieselsäureorganosols (a) sind an der Oberfläche organisch modifiziert. Besonders vorteilhaft ist dabei eine Modifizierung mit funktionellen und insbesondere mit polymerisationsfähigen Gruppen, wie Acrylat- oder Methacrylatgruppen, die nach der Polymerisation eines Soles (a), welches polymerisationsfähige Dispersionsmittel enthält, einen kovalenten Verbund der dispergierten SiO₂-Teilchen mit der polymeren Matrix ergeben.

Das in dem erfindungsgemäßen Dentalmaterial eingesetzte SiO₂-Sol (a) ist nach bekannten Verfahren und auch kommerziell erhältlich. So kann ein solches Sol aus käuflichen kolloidalen Lösungen von amorphem Siliciumdioxid in Wasser dadurch erhalten werden, daß zunächst die Oberfläche der SiO₂-Partikel durch Umsetzung, z.B. mit 3-(Meth)acryloyloxypropyltrialkoxysilan, modifiziert wird, anschließend das Wasser durch einen flüchtigen Alkohol, z.B. Isopropanol, ausgetauscht und der Alkohol durch das gewünschte Dispersionsmittel, z.B. 2-Hydroxyethylmethacrylat oder Triethylenglycoldimethacrylat, schließlich ersetzt wird. Besonders geeignete kommerziell erhältliche SiO₂-Sole werden unter der Bezeichnung Highlink®-OG von Sociètè Francaise Hoechst angeboten. In diesen nichtopaken SiO₂-Solen sind die SiO₂-Teilchen so oberflächenmodifiziert, daß sie mit verschiedenen Lösungs- und Dispersionsmitteln, wie Diolen, Hydroxy(meth)acrylaten oder Di(meth)acrylaten, verträglich sind. Erfindungsgemäß einsetzbare Typen sind dabei insbesondere Highlink®-OG 103-53, Highlink®-OG 100, Highlink®-OG 2-IV, Highlink®-OG 3-IV und Highlink®-OG 4-IV.

Es hat sich überraschenderweise herausgestellt, daß das erfindungsgemäße Dentalmaterial selbst bei Verwendung von SiO₂-Sol (a) mit einem sehr hohen SiO₂-Gehalt eine geringere Viskosität aufweist als Dentalmaterialien, die mit einer entsprechenden Menge an herkömmlicher Kieselsäure gefüllt sind, bei der die SiO₂-Teilchen jedoch in agglomerierter Form vorliegen.

Das erfindungsgemäße Dentalmaterial kann neben dem Kieselsäureorganosol (a) außerdem mindestens ein polymerisierbares organisches Bindemittel (b) enthalten. Dieses wird üblicherweise in einer Menge von 0 bis 80 Gew.-% und insbesondere 0 bis 50 Gew.-% in dem Dentalmaterial eingesetzt.

Als polymerisierbares organisches Bindemittel eignen sich alle für einen Dentalwerkstoff brauchbaren Bindemittel, insbesondere monofunktionelle oder polyfunktionelle (Meth)acrylate, die allein oder in Mischungen eingesetzt werden können. Bevorzugte Beispiele für diese Verbindungen sind Methyl(meth)acrylat, Isobutyl(meth)acrylat, Cyclohexyl(meth)acrylat, Tetraethylenglycoldi(meth)acrylat, Diethylenglycoldi(meth)acrylat, Ethylenglycoldi(meth)acrylat, Polyethylenglycoldi(meth)acrylat, Butandioldi(meth)acrylat, Hexandioldi(meth)acrylat, Decandioldi(meth)acrylat, Dodecandioldi(meth)acrylat, Bisphenol-A-di(meth)acrylat, 2,2-Bis-4-(3-(meth)acryloxy-2-hydroxy-propoxy)-phenylpropan (Bis-GMA) sowie die Produkte der Reaktion von Isocyanaten, insbesondere Diund/oder Triisocyanaten, mit OH-gruppenhaltigen (Meth)acrylaten. Besonders bevorzugte Beispiele für die zuletzt genannten Produkte sind durch Reaktion von 1 Mol Hexamethylendiisocyanat mit 2 Mol 2-Hydroxyethylmethacrylat und von 1 Mol Tri-(6-isocyanatohexyl)biuret mit 3 Mol 2-Hydroxyethyl(meth)acrylat erhältlich.

Besonders bevorzugte polymerisierbare organische Bindemittel (b) sind Triethylenglycoldimethacrylat, Bisphenol-A-Glycidylmethacrylat, Urethandimethacrylat aus 2,2,4-Trimethylhexamethylendiisocyanat (TMDI) und 2-Hydroxyethylmethacrylat (HEMA), Trimethylolpropantrimethacrylat oder Pentaerythritetramethacrylat.

Neben dem SiO₂-Sol (a) kann das erfindungsgemäße Dentalmaterial zudem auch herkömmliche anorganische oder organische teilchenförmige Füllstoffe (c) enthalten. Diese Füllstoffe (c) werden typischerweise in einer Menge von 0 bis 90 Gew.-%, insbesondere 0 bis 75 Gew.-%, in dem Dentalmaterial eingesetzt.

Beispiele für bevorzugte Füllstoffe (c) sind gefällte oder pyrogene Kieselsäuren, Calciumcarbonat, Caiciumhydroxyd, Glasfüller oder röntgenopake Stoffe, wie Ytterbiumfluorid, Bariumsulfat und Bariumhydroxyd.

Zur Erzielung einer verbesserten Haftung und Einbindung dieser herkömmlichen Füllstoffe werden sie mit Silanen, wie z.B. Methacryloyloxyalkylsilanen, z.B. dem käuflichen 3-Methacryloyloxypropyltrimethoxysilan, silanisiert.

Weiter kann das erfindungsgemäße Dentalmaterial ebenfalls (d) mindestens einen Polymerisationsinitiator und gegebenenfalls einen Beschleuniger enthalten.

Das erfindungsgemäße Dentalmaterial kann heiß, kalt oder durch Licht polymerisiert werden. Als Initiatoren für die Heißpolymerisation können die bekannten Peroxide wie Dibenzoylperoxid, Dilauroylperoxid, tert.-Butylperoctoat oder tert.-Butylperbenzoat eingesetzt werden. Darüber hinaus sind auch 2,2'-Azoisobuttersäurenitril (AIBN), Benzpinakol und 2,2'-Dialkylbenzpinakole geeignet.

Als Initiatoren für die Photopolymerisation können zum Beispiel Benzophenon und seine Derivate sowie Benzoin und seine Derivate verwendet werden. Weitere bevorzugte Photoinitiatoren sind die α-Diketone wie 9,10-Phenanthrenchinon, Diacetyl, Furil, Anisil, 4,4'-Dichlorbenzyl und 4,4'-Dialkoxybenzyl. Besonders bevorzugt wird Campherchinon verwendet. Darüber hinaus eignet sich auch die Gruppe der Acrylphosphinoxide gut zur Initiierung der Photopolymerisation. Zur Beschleunigung der Initiierung werden die Photoinitiatoren vorzugsweise zusammen mit einem Reduktionsmittel, besonders bevorzugt mit einem Amin, insbesondere einem aromatischen Amin, eingesetzt.

Als Initiatoren für die Kaltpolymerisation werden Radikale liefernde Redox-Systeme, zum Beispiel Benzoyl- oder Lauroylperoxid zusammen mit Aminen wie N,N-Dimethyl-p-toluidin, N,N-Dihydroxyethyl-p-toluidin oder anderen strukturverwandten Aminen eingesetzt.

Speziell bei Dentalmaterialien zur Zementierung von Dentalrestaurationen, wie beispielsweise Glaskeramik-Inlays, -Onlays, -Teilkronen und -Kronen, hat sich die Kombination von Photoinitiatoren mit unterschiedlichen Redoxsystemen bewährt. Bevorzugt sind Kombinationen aus Campherchinon, Benzoylperoxid und Aminen wie beispielsweise N,N-Dimethyl-p-toluidin und/oder N,N-Cyanoethylmethylanilin.

Die Konzentration der Initiatoren und Beschleuniger (d) liegt bevorzugt im Bereich von 0,05 bis 1,5 Gew.-%, besonders bevorzugt im Bereich von 0,2 bis 0,8 Gew.-%, bezogen auf die Menge der im Dentalmaterial eingesetzten Monomeren.

Es ist ebenfalls möglich, daß das erfindungsgemäße Dentalmaterial zumindest teilweise in polymerisierter Form vorliegt.

Besonders vorteilhaft wird das erfindungsgemäße Dentalmaterial als Füllungskomposit, Befestigungszement oder Adhäsiv eingesetzt. Dabei erweisen sich der verringerte Polymerisationsschrumpf und die verbesserten mechanischen Eigenschaften dieser Materialien als besonderer Vorteil.

Bei Verwendung des Dentalmaterials als Dentaladhäsiv wird das Sol von nicht-agglomerierten SiO₂-Teilchen (a) insbesondere mit häufig auch als Vernetzermonomere bezeichneten mehrfunktionellen polymerisierbaren organischen Dispersionsmitteln und/oder Bindemitteln, wie z.B. Triethylenglycoldimethacrylat, Bisphenol-A-Glycidylmethacrylat, Urethandimethacrylat aus HEMA und TMDI, kombiniert. Die erhaltenen erfindungsgemäßen Dentaladhäsive zeigen im Vergleich zu Systemen, die mit hochdisperser Kieselsäure gefüllt sind, eine deutlich geringere Viskosität, wobei als weitere Vorteile eine verringerte Abrasivität der Polymermatrix und eine Verringerung des Polymerisationsschrumpfes feststellbar sind.

Bei Verwendung des erfindungsgemäßen Dentalmaterials als Füllungskomposit werden neben dem Sol (a) und organischem Bindemittel (b) üblicherweise auch herkömmliche anorganische oder organische teilchenförmige Füllstoffe (c) eingesetzt. Dabei werden bei geringem Füllgrad Befestigungszemente und bei hohem Füllgrad Füllungsmaterialien erhalten. Darüber hinaus ist auch eine Verstärkung mit Fasern, z.B. Kurz- oder Langglasfasern sowie Cellulose- oder Polyamidfasern, möglich.

Schließlich kann das erfindungsgemäße Dentalmaterial auch noch übliche Hilfs- und Zusatzstoffe, wie Farbstoffe, Pigmente, Thixotropiehilfsmittel, Stabilisatoren z.B. Hydrochinonmonomethylether (MEHQ) oder 2,6-Di-tert.-butyl-4-methylphenol (BHT), Aromastoffe oder mikrobiocide Wirkstoffe enthalten.

Gegenstand der Erfindung ist auch die Verwendung des vorstehend definierten gefüllten und polymerisierbaren Materials zur Herstellung von Dentalmaterial. Wie oben erwähnt, zeichnet sich dieses Material durch einen Gehalt an (a) einem Sol von amorphen SiO₂-Teilchen in einem flüssigen, organischen Dispersionsmittel aus, wobei die SiO₂-Teilchen organisch oberflächenmodifiziert sind, eine mittlere Größe von 10 bis 100 nm aufweisen und nicht-agglomerisiert sind.

Im folgenden wird die Erfindung anhand von Beispielen näher erläutert.

### Beispiele

In den Beispielen werden folgende Stoffe eingesetzt:
- Monomere:: - Hexandioldiacrylat (HDDA),
- Triethylenglycoldimethacrylat (TEGDMA),
- Bisphenol-A-Glycidylmethacrylat (Bis-GMA),
- Urethandimethacrylat aus HEMA und TMDI, als UDMA bezeichnet.
- Kieselsäure-organosole (a):: - Highlink® OG 103-53 (Sociètè Francaise Hoechst): 50 Gew.-% SiO₂, 50 nm Partikelgröße, HDDA als Dispersionsmittel, Viskosität: 270 mPa•s/20°C,
- Highlink® OG 2-IV (Sociètè Francaise Hoechst): 51,7 Gew.-% SiO₂, 50 nm Partikelgröße, TEGDMA als Dispersionsmittel, Viskosität: 750 mPa•s/20°C,
- Highlink® OG 4-IV (Sociètè Francaise Hoechst): 48,7 Gew.-% SiO₂, 50 nm Partikelgröße, eine Mischung aus TEGDMA (47,0 Gew.-%), Bis-GMA (28,0 Gew.-%) und UDMA (25,0 Gew.-%) als Dispersionsmittel, Viskosität: 3,0 Pa•s/20°C;
- Herkömmliche Füllstoffe:: - Silanisiertes Bariumaluminiumsilikatglaspulver (Schott), Korngröße < 7 µm, als BaG bezeichnet,
- Ytterbiumfluorid (Rhone-Poulenc) (YbF₃),
- Sphärosil, SiO₂-ZrO₂-Mischoxid (Tokoyama Soda), Sekundärkorngröße < 7 µm;
- Photoinitiator + Beschleuniger:: - Mischung aus Campherchinon (CC) und N-(2-Cyanoethyl)-N-methylanilin (CEMA).

### Beispiel 1: Dentaladhäsiv mit Kieselsäureorganosol mit HDDA als Dispersionsmittel

Eine als Dentaladhäsiv einsetzbare transparente Mischung von 99,2 Gew.-% Highlink® OG 103-53 mit 0,3 Gew.-% CC und 0,5 Gew.-% CEMA sowie als Vergleich eine Mischung von 99,2 Gew.-% HDDA mit 0,3 Gew.-% CC und 0,5 Gew.-% CEMA wurden durch 6 Minuten lange Belichtung mit einer dentalen Strahlungsquelle (Spectramat, Firma Vivadent) polymerisiert. Der Polymerisationsschrumpf (ΔV) wurde aus der Differenz der gaspyknometrisch bestimmten Monomer- und Polymerdichte berechnet, und die Biegefestigkeit (BF) und der Biege-E-Modul (BEM) wurden nach der ISO-Norm 4049 (1988) bestimmt. Die Kugeldruckhärte (KDH) der Polymerisate wurde entsprechend der DIN 53456 (1973) gemessen.

| **Material mit** | **ΔV (Vol.-%)** | **BF (MPa)** | **BEM (GPa)** | **KDH (MPa)** |
|---|---|---|---|---|
| Highlink® OG 103-53 (Erfindung) | 7,5 | 44 | 2,73 | 149 |
| HDDA (Vergleich) | 13,1 | 39 | 1,73 | 74 |

### Beispiel 2: Dentaladhäsiv oder -lack mit Kieselsäureorganosolen mit verschiedenen Dispersionsmitteln

Analog zu Beispiel 1 wurden transparente Mischungen von (1) 99,2 Gew.-% Highlink® OG 2-IV oder (2) Highlink® OG 4-IV mit jeweils 0,3 Gew.-% CC und 0,5 Gew.-% CEMA sowie als Vergleichsbeispiele Mischungen von (3) 99,2 Gew.-% TEGDMA oder (4) einer Kombination aus TEGDMA (46,6 Gew.-%), Bis-GMA (27,8 Gew.-%) und UDMA (24,8 Gew.-%) mit jeweils 0,3 Gew.-% CC und 0,5 Gew.-% CEMA polymerisiert. Die erhaltenen Polymerisate hatten die folgenden Eigenschaften.

| **Nr.** | **Material mit** | **ΔV (Vol.-%)** | **BF (MPa)** | **BEM (GPa)** |
|---|---|---|---|---|
| 1. | Highlink® OG 2-IV (Erfindung) | 8,6 | 55 | 2,80 |
| 3. | TEGDMA (Vergleich) | 12,7 | 47 | 1,22 |
| 2. | Highlink® OG 4-IV (Erfindung) | 6,1 | 88 | 3,41 |
| 4. | TEGDMA/Bis-GMA/UDMA (Vergleich) | 8,0 | 83 | 1,97 |

Diese Ergebnisse zeigen, daß die erfindungsgemäßen Materialien im Vergleich zu Materialien ohne Kieselsäureorganosol eine Verringerung des Polymerisationsschrumpfes und eine Verbesserung der mechanischen Eigenschaften sowie der Materialhärte aufweisen, so daß sie sich besonders als Dentaladhäsive oder -lacke eignen.

Als weitere Vergleichsbeispiele hergestellte Mischungen der aufgeführten Monomerkomponenten mit herkömmlichen Füllstoffen zeigen, daß im Falle pyrogener Kieselsäure, z.B. OX-50, nur opake Mischungen geringer Durchärtungstiefe zugänglich sind oder im Falle von Fällungskieselsäure, z.B. HDK 2000 (Wacker-Chemie GmbH), höhere Füllungsgrade aufgrund der extremen Verdickungswirkung dieser Kieselsäure nicht erreichbar sind.

### Beispiel 3: Komposite von Kieselsäureorganosolen mit verschiedenen Dispersionsmitteln

Es wurden Kompositpasten M1 bis M6 in einem Planeten-Kneter (Linde) folgender Zusammensetzung (alle Angaben in Gew.-%) hergestellt.

| | **M-1** | **M-2** *** | **M-3** | **M-4** *** | **M-5** | **M-6** *** |
|---|---|---|---|---|---|---|
| Highlink® OG 103-53 | 24,0 | - | - | - | - | - |
| HDDA | - | 24,0 | - | - | - | - |
| Highlink® OG 2-IV | - | - | 26,0 | - | - | - |
| TEGDMA | - | - | - | 26,0 | - | 13,3 |
| Highlink® OG 4-IV | - | - | - | - | 28,4 | - |
| Bis-GMA | - | - | - | - | - | 7,9 |
| UDMA | - | - | - | - | - | 7,2 |
| BaG | 14,0 | 14,0 | 13,6 | 13,6 | 19,5 | 19,5 |
| Sphärosil | 13,5 | 13,5 | 13,1 | 13,1 | 18,9 | 18,9 |
| YbF₃ | 48,3 | 48,3 | 47,1 | 47,1 | 33,0 | 33,0 |
| CC + CEMA | 0,2 | 0,2 | 0,2 | 0,2 | 0,2 | 0,2 |

| | | | | | | |
|---|---|---|---|---|---|---|
| *** Vergleichsbeispiele | | | | | | |

Diese Pasten wurden dann 10 Minuten lang bei 200 mbar entlüftet und analog zu Beispiel 1 polymerisiert. Die erhaltenen Polymerisate hatten die folgenden Eigenschaften:

| **Material** | **ΔV (Vol.-%)** | **BF (MPa)** | **BEM (GPa)** |
|---|---|---|---|
| M-1 | 4,4 | 104 | 12,3 |
| M-2 (Vergleich) | 5,5 | 100 | 6,3 |
| M-3 | 2,4 | 123 | 13,6 |
| M-4 (Vergleich) | 6,2 | 117 | 6,4 |
| M-5 | 2,5 | 127 | 13,6 |
| M-6 (Vergleich) | 4,3 | 108 | 6,7 |

Diese Ergebnisse zeigen, daß die Verwendung der erfindungsgemäß eingesetzten Kieselsäureorganosolen als Komponente von dentalen Kompositen zu einer Verringerung der Schrumpfung und Verbesserung der mechanischen Eigenschaften führt.

## Patentansprüche

1. Gefülltes und polymerisierbares Dentalmaterial, **dadurch gekennzeichnet, daß** es
(a) ein Sol von amorphen SiO₂-Teilchen in einem flüssigen, organischen Dispersionsmittel enthält, wobei die SiO₂-Teilchen organisch oberflächenmodifiziert sind, eine mittlere Größe von 10 bis 100 nm aufweisen und nichtagglomeriert sind.

2. Dentalmaterial nach Anspruch 1, **dadurch gekennzeichnet, daß** es 1 bis 50 Gew.-% des Soles (a) enthält.

3. Dentalmaterial nach Anspruch 1 oder 2, **dadurch gekennzeichnet, daß** das Sol (a) 10 bis 70 und insbesondere 20 bis 55 Gew.-% SiO₂-Teilchen, bezogen auf das Sol, enthält.

4. Dentalmaterial nach einem der Ansprüche 1 bis 3, **dadurch gekennzeichnet, daß** das Dispersionsmittel mindestens eine polymerisierbare Verbindung enthält.

5. Dentalmaterial nach einem der Ansprüche 1 bis 4, **dadurch gekennzeichnet, daß** das flüssige, organische Dispersionsmittel
- mindestens ein Diol,
- mindestens ein Hydroxy(meth)acrylat,
- mindestens ein Di(meth)acrylat oder
- Mischungen der vorstehenden Verbindungen
enthält.

6. Dentalmaterial nach Anspruch 5, **dadurch gekennzeichnet, daß** das flüssige, organische Dispersionsmittel
- Hexandioldiacrylat,
- 2-Hydroxyethylmethacrylat,
- Triethylenglycoldimethacrylat,
- Bisphenol-A-Glycidylmethacrylat,
- ein Urethandimethacrylat oder
- Mischungen der vorstehenden Verbindungen
enthält.

7. Dentalmaterial nach einem der Ansprüche 1 bis 6, **dadurch gekennzeichnet, daß** es
(b) mindestens ein polymerisierbares organisches Bindemittel
enthält.

8. Dentalmaterial nach Anspruch 7, **dadurch gekennzeichnet, daß** das polymerisierbare organische Bindemittel (b)
- Triethylenglycoldimethacrylat,
- Bisphenol-A-Glycidylmethacrylat,
- das Urethandimethacrylat aus 2,2,4-Trimethylhexamethylendiisocyanat und 2-Hydroxyethylmethacrylat,
- Trimethylolpropantrimethacrylat und/oder
- Pentaerythrittetramethacrylat
ist.

9. Dentalmaterial nach einem der Ansprüche 1 bis 8, **dadurch gekennzeichnet, daß** es
(c) anorganische oder organische teilchenförmige Füllstoffe enthält.

10. Dentalmaterial nach einem der Ansprüche 1 bis 9, **dadurch gekennzeichnet, daß** es
(d) mindestens einen Polymerisationsinitiator und gegebenfalls einen Beschleuniger
enthält.

11. Dentalmaterial nach einem der Ansprüche 1 bis 10, **dadurch gekennzeichnet, daß** es ein Füllungskomposit, ein Befestigungszement oder ein Adhäsiv ist.

12. Dentalmaterial nach einem der Ansprüche 1 bis 11, **dadurch gekennzeichnet, daß** es in zumindest teilweise polymerisierter Form vorliegt.

13. Verwendung eines gefüllten und polymerisierbaren Materials, welches (a) ein Sol von amorphen SiO₂-Teilchen in einem flüssigen, organischen Dispersionsmittel enthält, wobei die SiO₂-Teilchen organisch oberflächenmodifiziert sind, eine mittlere Größe von 10 bis 100 nm aufweisen und nichtagglomeriert sind, zur Herstellung von Dentalmaterial.

## Claims

1. Filled and polymerizable dental material, **characterized in that** it contains
(a) a sol of amorphous SiO₂ particles in a liquid organic dispersion medium, wherein the SiO₂ particles are organically surface modified, have an average size of 10 to 100 nm and are non-agglomerated.

2. Dental material according to Claim 1, **characterized in that** it contains 1 to 50% by weight of the sol (a).

3. Dental material according to Claim 1 or 2, **characterized in that** the sol (a) contains 10 to 70% and especially 20 to 55% by weight of SiO₂ particles, based on the sol.

4. Dental material according to any of Claims 1 to 3, **characterized in that** the dispersion medium contains at least one polymerizable compound.

5. Dental material according to any of Claims 1 to 4, **characterized in that** the liquid organic dispersion medium contains
- at least one diol,
- at least one hydroxy(meth)acrylate,
- at least one di(meth)acrylate or
- mixtures of the above compounds.

6. Dental material according to Claim 5, **characterized in that** the liquid organic dispersion medium contains
- hexanediol diacrylate,
- 2-hydroxyethyl methacrylate,
- triethylene glycol dimethacrylate,
- bisphenol A glycidyl methacrylate,
- a urethane dimethacrylate or
- mixtures of the above compounds.

7. Dental material according to any of Claims 1 to 6, **characterized in that** it contains
(b) at least one polymerizable organic binder.

8. Dental material according to Claim 7, **characterized in that** the polymerizable organic binder (b) is
- triethylene glycol dimethacrylate,
- bisphenol A glycidyl methacrylate,
- the urethane dimethacrylate of 2,2,4-trimethylhexamethylene diisocyanate and 2-hydroxyethyl methacrylate,
- trimethylolpropane trimethacrylate and/or
- pentaerythritol tetramethacrylate.

9. Dental material according to any of Claims 1 to 8, **characterized in that** it contains
(c) inorganic or organic particulate fillers.

10. Dental material according to any of Claims 1 to 9, **characterized in that** it contains
(d) at least one polymerization initiator with or without an accelerant.

11. Dental material according to any of Claims 1 to 10, **characterized in that** it is a filling composite, a fixing cement or an adhesive.

12. Dental material according to any of Claims 1 to 11, **characterized in that** it is present in an at least partially polymerized form.

13. Use of a filled and polymerizable material which contains (a) a sol of amorphous SiO₂ particles in a liquid organic dispersion medium, wherein the SiO₂ particles are organically surface modified, have an average size of 10 to 100 nm and are non-agglomerated, for preparing dental material.

## Revendications

1. Matériau dentaire polymérisable et contenant une charge, **caractérisé en ce qu'**il contient
(a) un sol de particules de SiO₂ amorphe dans un agent dispersant organique liquide, les particules de SiO₂ étant modifiées en surface par des groupes organiques, ayant une taille moyenne de 10 à 100 nm et n'étant pas agglomérées.

2. Matériau dentaire selon la revendication 1, **caractérisé en ce qu'**il contient de 1 à 50 % en poids du sol (a).

3. Matériau dentaire selon la revendication 1 ou 2, **caractérisé en ce que** le sol (a) contient de 10 à 70 et en particulier de 20 à 55 % en poids de particules de SiO₂, par rapport au sol.

4. Matériau dentaire selon l'une quelconque des revendications 1 à 3, **caractérisé en ce que** l'agent dispersant contient au moins un composé polymérisable.

5. Matériau dentaire selon l'une quelconque des revendications 1 à 4, **caractérisé en ce que** l'agent dispersant organique liquide contient
- au moins un liant,
- au moins un hydroxy(méth)acrylate,
- au moins un di(méth)acrylate ou
- des mélanges des composés précédents.

6. Matériau dentaire selon la revendication 5, **caractérisé en ce que** l'agent dispersant organique liquide contient
- du diacrylate d'hexanediol,
- du méthacrylate de 2-hydroxyéthyle,
- du diméthacrylate de triéthylèneglycol,
- du méthacrylate de glycidyl-bisphénol A
- un uréthanne-diméthacrylate ou
- des mélanges des composés précédents.

7. Matériau dentaire selon l'une quelconque des revendications 1 à 6, **caractérisé en ce qu'**il contient
(b) au moins un liant organique polymérisable.

8. Matériau dentaire selon la revendication 7, **caractérisé en ce que** le liant organique polymérisable (b) est
- le diméthacrylate de triéthylèneglycol,
- le méthacrylate de glycidyl-bisphénol A,
- l'uréthanne-diméthacrylate obtenu à partir de 2,2,4-triméthylhexaméthylènediisocyanate et de méthacrylate de 2-hydroxyéthyle,
- le triméthacrylate de triméthylolpropane et/ou
- le tétraméthacrylate de pentaérythritol.

9. Matériau dentaire selon l'une quelconque des revendications 1 à 8, **caractérisé en ce qu'**il contient
(c) des charges organiques ou minérales particulaires.

10. Matériau dentaire selon l'une quelconque des revendications 1 à 9, **caractérisé en ce qu'**il contient
(d) au moins un initiateur de polymérisation et éventuellement un accélérateur.

11. Matériau dentaire selon l'une quelconque des revendications 1 à 10, **caractérisé en ce qu'**il contient un composite de plombage, un ciment de consolidation ou un adhésif.

12. Matériau dentaire selon l'une quelconque des revendications 1 à 11, **caractérisé en ce qu'**il se trouve sous forme au moins partiellement polymérisée.

13. Utilisation d'un matériau polymérisable et contenant une charge, qui contient (a) un sol de particules de SiO₂ amorphe dans un agent dispersant organique liquide, les particules de SiO₂ étant modifiées en surface par des groupes organiques, ayant une taille moyenne de 10 à 100 nm et n'étant pas agglomérées, pour la production de matériau dentaire.
